# EUROPEAN PATENT SPECIFICATION

(11) **EP 2 040 666 B1**
(45) Date of publication and mention of the grant of the patent: **20.01.2010**
(21) Application number: 07720225.7
(22) Date of filing: 16.07.2007
(51) Int. Cl.: A61K 8/49, A61Q 19/00

(54) **BENZIMIDAZOLES AS COOLING COMPOUNDS**
BENZIMIDAZOLE ALS KÜHLENDE VERBINDUNGEN
COMPOSÉS DE RAFRAÎCHISSEMENT À BASE DE BENZIMIDAZOLE

(30) Priority: 14.07.2006 US 830964 P
(43) Date of publication of application: 01.04.2009
(73) Proprietor: Givaudan SA, 1214 Vernier (CH)
(72) Inventor: FURRER, Stefan, Michael, Cincinnati, Ohio 45215 (US); BELL, Karen, Ann, Loveland, Ohio 45140 (US); GALOPIN, Christophe, Chesterfield, Virginia 23832 (US); MCCLUSKEY, Thomas, Scott, Amelia, Ohio 45102 (US); SLACK, Jay, Patrick, Loveland, Ohio OH 45140 (US)
(74) Representative: McStea, John Anthony
(86) International application number: PCT/CH2007/000340
(87) International publication number: WO 2008/006236

(56) References cited:
- EP-A- 1 493 336
- WO-A-2004/026840
- WO-A-2005/049553
- US-A- 4 296 093

## Description

Disclosed are cooling compounds, that is, compounds that give the sensation of cooling when applied to the skin or taken orally. Many such compounds are known to the art.

WO 2004/026840 discloses tetrahydropyrimidine-2-one derivatives for producing a cooling sensation.
WO 2005/049553 discloses N-substituted p-methane carboxamides for producing a cooling sensation.
EP 1493336 discloses mono methyl ester derivatives of dicarboxylic acids for producing a cooling sensation.
US 4296093 discloses alkyl-substituted cyclohexanamides for producing a cooling sensation.

There has been discovered a new class of cooling compounds. There is disclosed herein a non-therapeutic method of providing a cooling sensation to the skin or mucous membranes of the mouth by applying thereto a quantity of at least one chemical compound sufficient to cause a desirable degree of cooling sensation, the chemical compound comprising a compound of formula I: 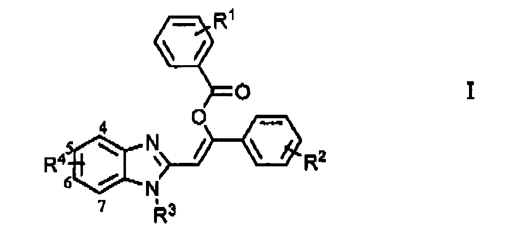
in which R¹, R² are independently in either the meta- or para-position and independently selected from the group consisting one of hydrogen, halide, C₁-C₃ alkyl (linear or branched), halide, C₁-C₃ alkoxy, nitro, nitrile, amide or ester;
R³ is selected from the group consisting of C₁-C₅ alkyl or C₁-C₅ alkenyl groups (linear or branched); and
R⁴ is selected from the group consisting of hydrogen, C₁-C₃ alkyl (linear or branched) or halide.

In certain embodiments, R¹, R² are independently selected from the group consisting of methoxy, chloro, bromo, fluoro, methyl, nitro or hydrogen.
In certain embodiments, R³ is selected from the group consisting of C₁-C₃ alkyl or C₁-C₃ alkenyl groups, in particular, *iso*-propyl, propyl, methyl or allyl.
In certain embodiments, R⁴ is selected from the group consisting of methyl, bromine, or hydrogen in position 6 or 5.

Some of the compounds hereinabove described are novel. There is therefore also provided a compound of the formula I 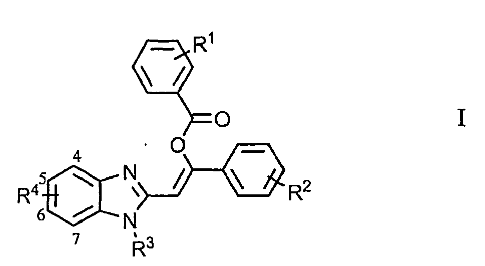 in which
R¹ and R² are independently in either the meta or para position and are selected from chloride and C₁-C₃ alkoxy groups,
R⁴ is C₁-C₃ alkyl (linear or branched), in certain embodiments methyl in position 5 or 6, and
R3 is C₁-C₃ alkyl, or C₁-C₃ alkenyl group, in certain embodiments *iso*-propyl, propyl, methyl or allyl.

In another embodiment with respect to the novel compounds, the alkoxy group from which R¹ and R² are selected is methoxy. In a further embodiment, R¹ and R² are either both methoxy, or one is methoxy and the other chloride.

The cooling compounds may be added to compositions applied to the skin or mucous membranes of the mouth to provide a cooling sensation. By "applying" is meant any form of non-therapeutically bringing into contact, for example, oral ingestion in solid, liquid or spray form, or, in the case of tobacco products, inhalation. In the case of application to the skin, it may be, for example, by including the compound in a cream or salve, or in a sprayable composition. There is therefore provided a composition for oral, nasal or topical application, comprising a cooling amount of at least one chemical compound as hereinabove described.

In the term "composition" is included any kind of commercial product in which the compounds hereinabove described may be desired to be used. In particular, there is provided a product selected from the group consisting of topical products, oral care products, nasal care products, toilet articles, ingestible products and chewing gum, which product comprises a product base and an effective amount of at least one cooling compound of formula (I).

Products include foodstuffs and beverages of all kinds; confectionery products, for example, candies, mints and gums; edible films of all kinds; medicinal preparations in solid, liquid or spray form for oral, nasal and topical use; toothpastes and tooth gels; mouthwashes; skin lotions; cosmetic creams; tobacco products and aerosol products. Apart from the cooling compounds described herein, such products are otherwise entirely conventional in their formulation, and all of the known standard ingredients may be used in art-recognised quantities. Thus, for example, in the case of a medicinal product, the ingredients is that substance, or are those substances, that confer the particular medicinal nature of the product on it, and are selected from the substances known to provide the desired medicinal effect.

In addition, all the normal auxiliary ingredients needed to make a commercial product in a desired form meay be used. Examples of such ingredients include pigments, dyestuffs and coloring matters; surfactants and emulsifiers; rheology-modifying agents; thickening agents; fillers and extenders; solvents and diluents; antioxidants; preservative materials, and foam stabliizers.

The incorporation of the cooling compounds in such products is also entirely conventional and can be achieved by the standard methods of the art. Thus, for example, they can be incorporated by mixing directly into a product base, or into a premix, which is later incorporated into a product. They may also be incorporated by any kind of encapsulation method and utilising any of the known and commercially-successful technologies, for example, granulation, spray-drying, coating, and coacervation. Any suitable encapsulation material may be used.

The subject compounds and method of use give a superior cooling effect, at least comparable with the best commercial cooling agents. It is of course possible and permissible to blend two or more subject compounds. In addition, conventional cooling agents known to the art may also be used in conjunction with the compositions described above. These include menthol, menthone, isopulegol, N-ethyl p-menthanecarboxamide (WS-3), N,2,3-trimethyl-2-isopropylbutanamide (WS-23), menthyl lactate, menthone glycerine acetal (Frescolat® MGA), mono-menthyl succinate (Physcool®), mono-menthyl glutarate, O-menthyl glycerine (CoolAct® 10) and 2-sec-butylcyclohexanone (Freskomenthe®).

The subject compounds and methods are now further described with reference to the following examples.

### Example 1

### General Preparation:

### A) Preparation of 2-methyl-1-alkyl 1H-benzo[d]imidazole, example 2-methyl-1-propyl-1H-benzo[d]imidazole:

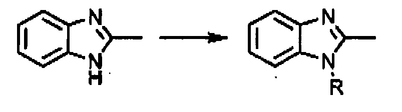

In a 500mL flask, fitted with magnetic stirrer, 33g of potassium hydroxide pellets (86%) [0.50 mol], 13.22g methylbenzimidazole and 250mL of acetone were added. 20g of propyliodide was then added and the mixture was stirred at room temperature for 3h. The mixture was extracted with 2x methyl tert.-butyl ether and water. The org. layers were washed with brine, dried over MgSO₄ and concentrated. The crude product purified by column chromatography yields 15.3g of brown oil (88% yield).
¹H NMR (300 MHz; CDCl₃) δ: 7.7 (m, 1H) 7.2 (m, 3H), 4.04 (dd, 2H), 2.60 (d, 3H), 1.83 (m, 2H), 0.96 (dd, 3H)
¹³C NMR (300 MHz; CDCl₃) δ: 151.4, 142.7, 135.2, 121.8, 121.6, 119.0, 109.2, 45.3, 23,0, 13.9,11.4

### 2-methyl-1-isopropyl-1H-benzo[d]imidazole:

¹H NMR (300 MHz; CDCl₃) δ: 7.7-7.5 (m, 1H) 7.2 (m, 3H), 4.65 (m, 1H), 2.61 (s, 3H), 1.62 (m, 6H)
¹³C NMR (300 MHz; CDCl₃) δ: 150-9, 143.1, 133.8, 121.8, 121.5, 119.2, 111.1, 48.0, 21.4, 15.0, 14.9

### B) Preparation of ketols from 1-alkyl-2-methyl-1H-benzo[d]imidazoles:

2-methyl-1-isopropyl-1H-benzo[d]imidazole was added to a round bottom flask with a N₂ inlet, magnetic stirring bar and a thermocouple and dissolved in THF (4 mL/mmol). The resulting mixture was cooled to -78°C using dry ice/isopropanol bath and Lithium diisopropylamide (2M solution in THF/n-heptane) (1.1 eq.) was added slowly using a syringe while maintaining the temperature <-60°C. The reddish reaction mixture was allowed to stir at -78°C for about 2h and a solution of methyl benzoate (1.5 eq) in THF (1mL/mmol) was added slowly over a period of 10min, using a syringe. The yellowish colored solution was slowly allowed to warm to room temperature and the progress of the reaction was monitored by TLC. After the reaction was complete, the mixture was cooled using ice/water bath and saturated aqueous NH₄Cl (4mL/mmol) and MTBE (4mL/mmol) was added. The organic layer was separated, washed with water and concentrated under vacuum at -35 oC. The residue was purified on silica gel using ethyl acetate/heptane.

### C) Preparation of benzimiazole-based analogs:

Ketol was added to a round bottom flask with a N₂ inlet, magnetic stirring bar and a thermocouple and dissolved in toluene (5mL/mmol). To the mixture triethylamine (1.5 eq) was added. The resulting mixture was cooled to 0°C using ice/water bath and benzoyl chloride (1.2 eq.) was added slowly using a syringe while maintaining internal temperature <10°C. The dark colored solution was slowly allowed to warm to room temperature and the progress of the reaction was monitored by TLC. After the reaction was complete, the mixture was cooled using ice/water bath and saturated aqueous NaHCO₃ was added. The organic layer was separated, washed with water and concentrated under vacuum at ∼35°C. The residue was stirred with ethyl acetate/heptane to give a solid suspension. The solid was collected by filtration and washed with heptane and dried.

### Examples 2-12

Using the method described in Example 1, a number of compounds were prepared.

### Example 2

### 1-(4-chlorophenyl)-2-(1-isopropyl-5-methyl-1H-benzo[d]imidazol-2-yl)vinyl 4-methoxybenzoate

¹H NMR (300 MHz; CDCl₃) δ: 8.2 (d, 2H) 7.6 (d 2H), 7.4-7.3 (m, 4H), 7.0 (m, 4H), 4.9 (m, 1H), 3.9 (s, 3H). 2.4 (s, 3H), 1.6 (d, 6H)
¹³C NMR (300 MHz; CDCl₃) δ: 164.0, 163.8, 145.9, 135.7, 133, 132.7, 131.9, 130.5, 128.9, 126.7, 124.2, 121.8, 119.4, 113.7, 111.1, 103.6, 55.5, 48.4, 21.7, 21.3
LC-MS (ESI+), m/z 461 [M+]
MP: 155-160°C

### Example 3

### 2-(1-isopropyl-5-methyl-1H-benzo[d]imidazol-2-yl)-1-(4-methoxyphenyl)vinyl 4-methoxybenzoate

¹H NMR (300 MHz; CDCl₃) δ: 8.3-8.1 (m, 2H) 7.7-7.5 (m 2H), 7.4-7.2 (m, 2H), 7.1-6.8 (m, 6H), 5.0-4.8 (m, 1H), 4.0-3.6 (m, 6H). 2.6-2.2 (m, 3H), 1.8-1.4 (m, 6H)
¹³C NMR (300 MHz; CDCl₃) δ: 164.1, 163.6, 160.8, 152.3, 145.7, 132.5, 131.3, 130.8, 127.2, 126.9, 123.9, 121.7, 122.0, 119.5, 114.1, 113.6, 110.8, 108.4, 101.4, 55.4, 55.3, 48.1, 21.6, 21.2
LC-MS (ESI+), m/z 457 [M+]
MP: 147-152°C

### Example 4

### 1-(4-chlorophenyl)-2-(1-isopropyl-6-methyl-1H-benzo[d]imidazol-2-yl)vinyl 4-chlorobenzoate

¹H NMR (300 MHz; CDCl₃) δ: 8.3 (d, 2H) 7.6 (d 2H), 7.5-7.4 (m, 4H), 7.2-7.0 (m, 4H), 5.1-5.0 (m, 1H), 2.4 (s, 3H), 1.7 (d, 6H)
¹³C NMR (300 MHz; CDCl₃) δ: 163.3, 161.2, 144.2, 141.3, 140.3, 136.1, 134.2, 132.1, 131.8, 129.3, 128.9, 128.7, 127.1, 126.7, 125.4, 117.4, 112.0, 101.3, 49.6, 21.9, 21.5
LC-MS (ESI+), m/z 465 [M+]
MP: 158-165°C

### Example 5

### 2-(1-isopropyl-6-methyl-1H-benzo[d]imidazol-2-yl)-1-(4-methoxyphenyl)vinyl 4-methoxybenzoate

¹H NMR (300 MHz; CDCl₃) δ: 8.2 (d, 2H) 7.6 (d 2H), 7.4 (d, 1H), 7.0-6.8 (m, 7H), 5.0-4.8 (m, 1H), 3.9 (s, 3H), 3.8 (s, 3H), 2.4 (s, 3H), 1.7 (d, 6H)
¹³C NMR (300 MHz; CDCl₃) δ: 164.2, 163.6, 160.8, 152.5, 146.3, 132.9, 132.6, 132.1, 127.1, 126.9, 123.4, 122.1, 119.2, 114.1, 113.5, 111.2, 101.3, 55.4, 55.2, 48.1, 21.9, 21.6
LC-MS (ESI+), m/z 457 [M+]
MP: 125-130°C

### Example 6

### 1-(4-chlorophenyl)-2-(1-propyl-1H-benzo[d]imidazol-2-yl)vinyl 4-chlorobenzoate

¹H NMR (300 MHz; DMSO) δ: 8.2 (d, 2H) 7.9 (d 2H), 7.7 (d, 2H), 7.6-7.5 (m, 3H), 7.4 (s, 1H), 7.2-7.1 (m, 2H), 4.4 (t, 2H), 1.9-1.7 (m, 2H), 0.9 (t, 3H)

### Example 7

### 1-(4-fluorophenyl)-2-(1-methyl-1H-benzo[d]imidazol-2-yl)vinyl 4-fluorobenzoate

¹H NMR (300 MHz; DMSO) δ: 8.3-8.2 (m, 2H) 7.9 (m 2H), 7.5 (d, 1H), 7.4 (t, 2H), 7.35 (t, 1H), 7.3 (t, 2H), 7.2 (t, 1H), 7.1-7.0 (m, 2H), 3.95 (s, 3H)

### Example 8

### 2-(1-methyl-1H-benzo[d]imidazol-2-yl)-1-p-tolylvinyl 4-methylbenzoate

¹H NMR (300 MHz; DMSO) δ: 8.05 (d, 2H) 7.75 (d, 2H), 7.5 (d, 1H), 7.45 (d, 2H), 7.3 (m, 3H), 7.25-7.05 (m, 3H), 3.95 (s, 3H), 2.5 (s, 3H), 2.4 (s, 3H)

### Example 9

### 1-(4-chlorophenyl)-2-(1-isopropyl-1H-benzo[d]imidazol-2-yl)vinyl 4-chlorobenzoate

¹H NMR (300 MHz; DMSO) δ: 8.15 (d, 2H) 7.9 (d, 2H), 7.7 (t, 3H), 7.55 (d, 2H), 7.5 (s, 1H), 7.2-7.05 (m, 3H), 5.2 (m, 1H), 1.6 (d, 6H)

### Example 10

### 1-(4-chlorophenyl)-2-(1-isopropyl-5-methyl-1H-benzo[d]imidazol-2-yl)vinyl 4-chlorobenzoate

¹H NMR (300 MHz; CDCl₃) δ: 8.2 (d, 2H), 7.6(d 2H), 7.5 (d, 2H), 7.4 (m, 3H), 7.2 (s, 1H), 7.0 (d, 1H), 6.9 (s, 1H), 4.9-4.8 (m, 1H), 2.4 (s, 3H), 1.7 (d, 6H)
¹³C NMR (300 MHz; CDCl₃) δ: 163.8, 150.6, 145.9, 139.6, 135.6, 133.1, 131.8, 131.5, 130.9, 129.2, 128.9, 128.6, 128.3, 126.5, 124.0, 119.8, 110.8, 103.6, 47.9, 21.7, 21.2
MP: 145-152°C

### Example 11

### 2-(1-isopropyl-5-methyl-1H-benzo[d]imidazol-2-yl)-1-(4-methoxyphenyl)vinyl 4-chlorobenzoate

¹H NMR (300 MHz; CDCl₃) δ: 8.3 (d, 2H), 7.6(d 2H), 7.5 (m, 2H), 7.3 (m, 2H), 7.1-7.0 (m, 2H), 6.8 (d, 2H), 5.0-4.9 (m, 1H), 3.7 (s, 3H), 2.4 (s, 3H), 1.7 (d, 6H)
¹³C NMR (300 MHz; CDCl₃) δ: 163.3, 161.1, 145.7, 139.9, 132.0, 131.7, 130.0, 129.2, 128.7, 127.8, 127.0, 126.1, 124.5, 116.5, 114.8, 111.2, 109.0, 55.2, 48.7, 21.5, 21.2
MP: 160-166°C

### Example 12

### 1-(4-chlorophenyl)-2-(1-isopropyl-6-methyl-1H-benzo[d]imidazol-2-yl)vinyl 4-methoxybenzoate

¹H NMR (300 MHz; CDCl₃) δ: 8.3 (d, 2H), 7.6(m, 3H), 7.5 (d, 1H), 7.2 (s, 1H), 7.1 (m, 3H), 7.0 (d, 2H), 5.2-5.0 (m, 1H), 3.8 (s, 3H), 2.4 (s, 3H), 1.7 (d, 6H)
¹³C NMR (300 MHz; CDCl₃) δ: 164.1, 163.6, 154.2, 144.2, 136.2, 134.5, 133.1, 131.6, 128.7, 126.9, 125.8, 120.5, 117.0, 113.9, 112.2, 101.5, 55.4, 50.1, 21.9, 21.5

### Example 13

### Experiments on cooling properties/intensities.

A group of panelists was asked to taste various aqueous solutions of compounds of formula (I) and to indicate which solutions had cooling intensities similar or slightly higher than that of a solution of menthol at 2ppm. The results are shown in Table 1.

**Table 1: Experiments on cooling intensity**

| Chemical | Concentration | Odor |
|---|---|---|
| Comparison: 1-Menthol | 2ppm solution | Minty |
| N-ethyl p-menthanecarboxamide (WS-3) | 1.5 ppm | None |
| 1-(4-methoxyphenyl)-2-(1-methyl-1H-benzo[d]imidazol-2-yl)vinyl 4-methoxybenzoate | 0.5ppm | None |
| 2-(1-isopropyl-6-methyl-1H-benzo[d]imidazol-2-yl)-1-(4-methoxyphenyl)vinyl 4-methoxybenzoate | 0.5ppm | None |
| (Z)-2-(1-isopropyl-5-methyl-1H-benzo[d]imidazol-2-yl)-1-(4-methoxyphenyl)vinyl 4-methoxybenzoate | 0.5ppm | None |
| (E)-2-(1-methyl-1H-benzo[d]imidazol-2-yl)-1-p-tolylvinyl 4-methylbenzoate | 2.0ppm | None |

### Example 14

### Application in toothpaste

| | |
|---|---|
| opaque toothgel | 99.50 g |
| cooling compound* as a 5% (w/w)solution in ethanol | 0.20g |
| peppermint oil, terpeneless | 0.25g |
| saccharin | 0.20g |

| | |
|---|---|
| *Compound of Example 5 | |

The chemicals were mixed in the toothgel, a piece of toothgel was put on a toothbrush and a panelist's teeth were brushed. The mouth was rinsed with water and the water was spit out. An intense cooling sensation was felt by the panelist in all areas of the mouth. The cooling perception lasted for 90 minutes.

Although the invention has been described in detail through the above detailed description and the preceding examples, these examples are for the purpose of illustration only and it is understood that variations and modifications can be made by one skilled in the art.

## Claims

1. A non-therapeutic method of providing a cooling sensation to the skin or mucous membranes of the mouth by applying thereto a quantity of at least one chemical compound sufficient to cause a desirable degree of cooling sensation, the chemical compound comprising a compound of formula I: 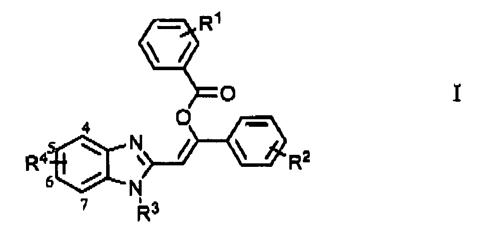
in which R¹, R² are independently in either the meta- or para-position and independently selected from the group consisting of hydrogen, halide, C₁-C₃ alkyl (linear or branched), C₁-C₃ alkoxy, nitro, nitrile, amide or ester;
R³ is selected from the group consisting of C₁-C₅ alkyl or C₁-C₅ alkenyl groups (linear or branched); and
R⁴ is selected from the group consisting of hydrogen, C₁-C₃ alkyl (linear or branched), or a halide.

2. The method according to claim 1, in which R¹, R² independently are selected from the group consisting of methoxy, chloro, bromo, fluoro, methyl, nitro or hydrogen.

3. The method according to claim 1, in which R³ is selected from the group consisting of C₁-C₃ alkyl or C₁-C₅ alkenyl groups.

4. The method according to claim 3, in which R³ is selected from the group consisting of *iso*-propyl, propyl, methyl or allyl.

5. The method according to claim 1, in which R⁴ is located in either position 5 or 6 and is selected from the group consisting of methyl, bromine or hydrogen.

6. A compound of the formula I 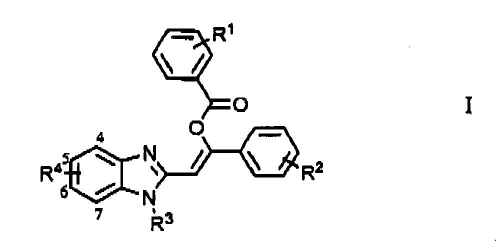 in which
R¹ and R² are independently in either the meta- or para-position and are selected from chloride and C₁-C₃ alkoxy groups,
R⁴ is C₁-C₃ alkyl (linear or branched), and
R3 is C₁-C₃ alkyl or C₁-C₅ alkenyl group.

7. A composition for oral, nasal or topical application comprising a cooling amount of at least one chemical compound comprising a compound of formula I: 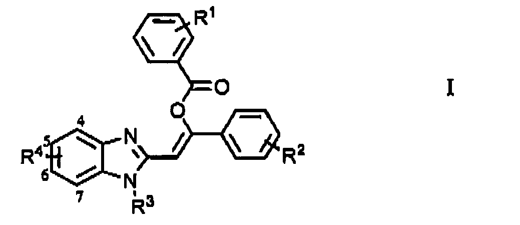
in which R¹, R² are independently in either the meta- or para-position and independently selected from the group consisting of hydrogen, halide, C₁-C₃ alkyl (linear or branched), C₁-C₃ alkoxy, nitro, nitrile, amide or ester;
R³ is selected from the group consisting of C₁-C₅ alkyl or C₁-C₅ alkenyl groups (linear or branched); and
R⁴ is selected from the group consisting of hydrogen, C₁-C₃ alkyl (linear or branched) or halide.

8. The composition according to claim 7, in which R¹, R² independently are selected from the group consisting of methoxy, chloro, bromo, fluoro, methyl, nitro or hydrogen.

9. The composition according to claim 7, in which R³ is selected from the group consisting of C₁-C₃ alkyl or C₁-C₅ alkenyl groups.

10. The composition according to claim 9, in which R³ is selected from the group consisting of *iso*-propyl, propyl, methyl or allyl.

11. The composition according to claim 7, in which R⁴ is located in either position 5 or 6 and is selected from the group consisting of methyl, bromine or hydrogen.

## Patentansprüche

1. Nichttherapeutisches Verfahren zur Bereitstellung eines kühlenden Gefühls auf der Haut oder den Schleimhäuten des Munds durch Aufbringen einer Menge mindestens einer chemischen Verbindung, die zur Bewirkung eines wünschenswerten Grads von kühlendem Gefühl ausreicht, wobei die chemische Verbindung eine Verbindung der Formel I: 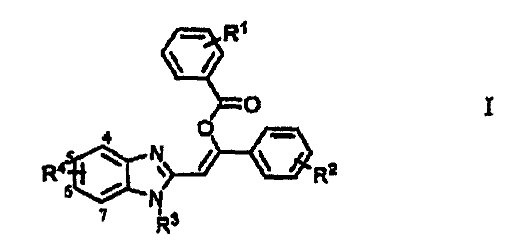
worin R¹ und R² unabhängig voneinander entweder in meta- oder in para-Position stehen und unabhängig voneinander aus der Gruppe bestehend aus Wasserstoff, Halogenid, C₁-C₃-Alkyl (linear oder verzweigt), C₁-C₃-Alkoxy, Nitro, Nitril, Amid oder Ester ausgewählt sind;
R² aus der Gruppe bestehend aus C₁-C₅-Alkyl- oder C₁-C₅-Alkenylgruppen (linear oder verzweigt) ausgewählt ist und
R⁴ aus der Gruppe bestehend aus Wasserstoff, C₁-C₃-Alkyl (linear oder verzweigt) oder Halogenid ausgewählt ist;
umfaßt.

2. Verfahren nach Anspruch 1, bei dem R¹ und R² unabhängig voneinander aus der Gruppe bestehend aus Methoxy, Chlor, Brom, Fluor, Methyl, Nitro oder Wasserstoff ausgewählt sind.

3. Verfahren nach Anspruch 1, bei dem R³ aus der Gruppe bestehend aus C₁-C₃-Alkyl- oder C₁-C₅-Alkenylgruppen ausgewählt ist.

4. Verfahren nach Anspruch 3, bei dem R³ aus der Gruppe bestehend aus Isopropyl, Propyl, Methyl oder Allyl ausgewählt ist.

5. Verfahren nach Anspruch 1, bei dem R⁴ entweder in 5- oder in 6-Position steht und aus der Gruppe bestehend aus Methyl, Brom oder Wasserstoff ausgewählt ist.

6. Verbindung der Formel I: 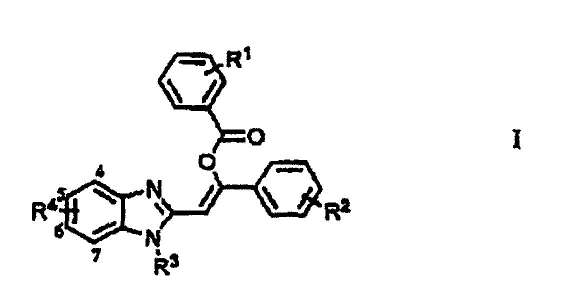
worin R¹ und R² unabhängig voneinander entweder in meta- oder in para-Position stehen und unter Chlorid und C₁-C₃-Alkoxygruppen ausgewählt sind;
R⁴ für C₁-C₃-Alkyl (linear oder verzweigt) steht und
R³ für eine C₁-C₃-Alkyl- oder C₁-C₅-Alkenylgruppe steht.

7. Zusammensetzung für die orale, nasale oder topische Anwendung, umfassend eine kühlende Menge mindestens einer chemischen Verbindung, die eine Verbindung der Formel I: 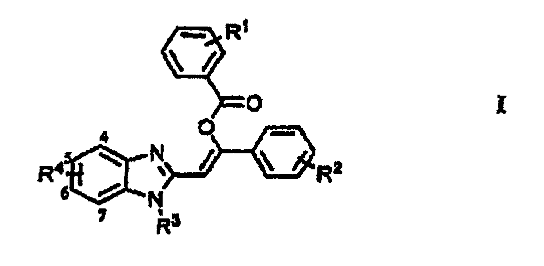
worin R¹ und R² unabhängig voneinander entweder in meta- oder in para-Position stehen und unabhängig voneinander aus der Gruppe bestehend aus Wasserstoff, Halogenid, C₁-C₃-Alkyl (linear oder verzweigt), C₁-C₃-Alkoxy, Nitro, Nitril, Amid oder Ester ausgewählt sind;
R³ aus der Gruppe bestehend aus C₁-C₅-Alkyl- oder C₁-C₅-Alkenylgruppen (linear oder verzweigt) ausgewählt ist und
R⁴ aus der Gruppe bestehend aus Wasserstoff, C₁-C₃-Alkyl (linear oder verzweigt) oder Halogenid ausgewählt ist;
umfaßt.

8. Zusammensetzung nach Anspruch 7, bei der R¹ und R² unabhängig voneinander aus der Gruppe bestehend aus Methoxy, Chlor, Brom, Fluor, Methyl, Nitro oder Wasserstoff ausgewählt sind.

9. Zusammensetzung nach Anspruch 7, bei der R³ aus der Gruppe bestehend aus C₁-C₃-Alkyl- oder C₁-C₅-Alkenylgruppen ausgewählt ist.

10. Zusammensetzung nach Anspruch 9, bei der R³ aus der Gruppe bestehend aus Isopropyl, Propyl, Methyl oder Allyl ausgewählt ist.

11. Zusammensetzung nach Anspruch 7, bei der R⁴ entweder in 5- oder in 6-Position steht und aus der Gruppe bestehend aus Methyl, Brom oder Wasserstoff ausgewählt ist.

## Revendications

1. Méthode non thérapeutique d'obtention d'une sensation rafraîchissante sur la peau ou les muqueuses de la bouche en y appliquant une quantité d'au moins un composé chimique suffisante pour provoquer un degré désirable de sensation rafraîchissante, le composé chimique comprenant un composé de formule I : 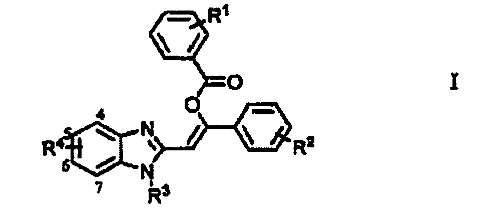
dans laquelle R¹, R² sont indépendamment soit en position méta soit en position para et sont choisis indépendamment dans le groupe constitué d'hydrogène, d'halogénure, de C₁-C₃ alkyle (linéaire ou ramifié), de C₁-C₃ alcoxy, de nitro, de nitrile, d'amide ou d'ester ;
R³ est choisi dans le groupe constitué de groupements C₁-C₅ alkyle ou C₁-C₅ alcényle (linéaires ou ramifiés) ; et
R⁴ est choisi dans le groupe constitué d'hydrogène, de C₁-C₃ alkyle (linéaire ou ramifié), ou d'halogénure.

2. Méthode selon la revendication 1, **caractérisée en ce que** R¹, R² indépendamment sont choisis dans le groupe constitué de méthoxy, de chloro, de bromo, de fluoro, de méthyle, de nitro ou d'hydrogène.

3. Méthode selon la revendication 1, **caractérisée en ce que** R³ est choisi dans le groupe constitué de groupements C₁-C₃ alkyle ou C₁-C₅ alcényle.

4. Méthode selon la revendication 3, **caractérisée en ce que** R³ est choisi dans le groupe constitué d'*iso*-propyle, de propyle, de méthyle ou d'allyle.

5. Méthode selon la revendication 1, **caractérisée en ce que** R⁴ est situé soit en position 5 soit en position 6 et est choisi dans le groupe constitué de méthyle, de brome ou d'hydrogène.

6. Composé de formule I 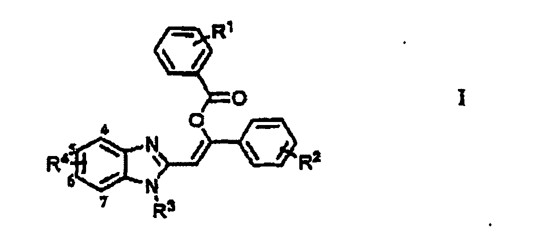 dans laquelle
R¹, R² sont indépendamment soit en position méta soit en position para et sont choisis parmi des groupements chlorure et C₁-C₃ alcoxy,
R⁴ est C₁-C₃ alkyle (linéaire ou ramifié), et
R³ est un groupement C₁-C₃ alkyle ou C₁-C₅ alcényle.

7. Composition destinée à l'application par voie orale, nasale ou topique, comprenant une quantité rafraîchissante d'au moins un composé chimique comprenant un composé de formule I : 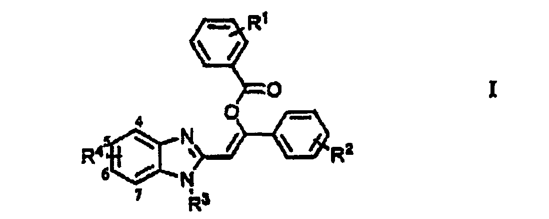
dans laquelle R¹, R² sont indépendamment soit en position méta soit en position para et sont choisis indépendamment dans le groupe constitué d'hydrogène, d'halogénure, de C₁-C₃ alkyle (linéaire ou ramifié), de C₁-C₃ alcoxy, de nitro, de nitrile, d'amide ou d'ester ;
R³ est choisi dans le groupe constitué de groupements C₁-C₅ alkyle ou C₁-C₅ alcényle (linéaires ou ramifiés) ; et
R⁴ est choisi dans le groupe constitué d'hydrogène, de C₁-C₃ alkyle (linéaire ou ramifié), ou d'halogénure.

8. Composition selon la revendication 7, **caractérisée en ce que** R¹, R² indépendamment sont choisis dans le groupe constitué de méthoxy, de chloro, de bromo, de fluoro, de méthyle, de nitro ou d'hydrogène.

9. Composition selon la revendication 7, **caractérisée en ce que** R³ est choisi dans le groupe constitué de groupements C₁-C₃ alkyle ou C₁-C₅ alcényle.

10. Composition selon la revendication 9, **caractérisée en ce que** R³ est choisi dans le groupe constitué d'*iso*-propyle, de propyle, de méthyle ou d'allyle.

11. Composition selon la revendication 7, **caractérisée en ce que** R⁴ est situé soit en position 5 soit en position 6 et est choisi dans le groupe constitué de méthyle, de brome ou d'hydrogène.
